(19) Europäisches Patentamt
European Patent Office
Office européen des brevets

(11) **EP 0 495 039 B2**

(12) **NEW EUROPEAN PATENT SPECIFICATION**

(45) Date of publication and mention
of the opposition decision:
**08.10.2003 Bulletin 2003/41**

(45) Mention of the grant of the patent:
**15.10.1997 Bulletin 1997/42**

(21) Application number: **91913688.7**

(22) Date of filing: **05.08.1991**

(51) Int Cl.$^7$: **C01B 33/193**, A61K 7/16

(86) International application number:
**PCT/GB91/01331**

(87) International publication number:
**WO 92/002454 (20.02.1992 Gazette 1992/05)**

(54) **SILICAS**

KIESELSÄUREN

SILICES

(84) Designated Contracting States:
**AT BE CH DE DK ES FR GB GR IT LI NL SE**

(30) Priority: **06.08.1990 EP 90308608**

(43) Date of publication of application:
**22.07.1992 Bulletin 1992/30**

(73) Proprietor: **INEOS Silicas Limited
Warrington WA5 1AB (GB)**

(72) Inventors:
• **McKEOWN, Ian, Patrick
Liverpool L19 3QZ (GB)**

• **STANIER, Peter, William
Sand Bach, Cheshire CW11 9EZ (GB)**

(74) Representative:
**Collingwood, Anthony Robert et al
LLoyd Wise, McNeight & Lawrence,
Regent House
Heaton Lane
Stockport, Cheshire SK4 1BS (GB)**

(56) References cited:
EP-A- 0 227 334      EP-A- 0 308 165
EP-A- 0 315 503      DE-A- 2 446 038
US-A- 3 864 470

EP 0 495 039 B2

**Description**

<u>Field of the invention</u>

This invention relates to synthetic precipitated silicas of use, for example, as abrasive agents in toothpaste compositions.

<u>Background to the invention</u>

Toothpaste compositions are well characterised in the literature and many compositions are disclosed in patent specifications and other literature. Toothpaste compositions contain a number of specific components for example abrasive agents, fluoride sources, binders, preservatives, humectants, anti plaque agents, colouring agents, water, flavour and other optional ingredients. Of these components the abrasive agent is required to provide the appropriate cleaning and plaque removal without subjecting the tooth itself to excessive abrasion. Typically a toothpaste composition will contain from about 5% to about 50%, preferably up to about 30%, by weight of abrasive. Commonly used abrasives are aluminas, calcium carbonates and calcium phosphates.

More recently synthetic silicas have been adopted because of their efficient cleaning, compatibility with other ingredients and their physical properties. Increasingly the sources of fluoride, e.g. sodium fluoride and stannous fluoride, are more sensitive to the presence of trace amounts of cations, e.g. calcium and magnesium, which are commonly found in silicas due to their processing.

An important property of a silica for use in transparent toothpaste formulations is refractive index, and it has been shown this property can be controlled by careful selection of process conditions in the preparation of the silica.

<u>General description of the invention</u>

The amorphous precipitated silicas of the invention have a structure defined by physical characteristics which allow the silicas to be used in transparent toothpastes with a considerable degree of formulation flexibility and provide a medium degree of abrasivity and optionally relatively small degree of thickening even at high, i.e. 20% w/w, loadings. However silicas of the invention with higher oil absorptions, i.e. 140 to 160 $cm^3$/100g, illustrated by Example 9, are able to provide thickening of the composition at high loadings. This may allow a thickening agent, e.g. a silica, to be used at a reduced level in the composition. The silicas can be prepared with low levels of cations, e.g. calcium, magnesium and aluminium, by washing the filter cake with de-ionised water such that the dry product subsequently gives extra stability when formulated in to a toothpaste containing fluoride ions.

The invention provides an amorphous precipitated silica having i) a surface area in the range from 250, preferably from about 300, more preferably from about 350, to 600$m^2$/g, preferably not more than about 550$m^2$/g, ii) an oil absorption (using linseed oil) of 90, preferably from about 110, to 160 $cm^3$/100g, preferably not more than about 140 $cm^3$/100g iii) a weight mean particle size in the range 5 microns to 20 microns, preferably up to about 15 microns, iv) a plastic abrasion value (PAV) in the range from about 8 to about 14, preferably at least about 10, v) a transmission of at least 80%, within the refractive index range of 1.435 to 1.444 and vi) a mean pore diameter in the range from 1.5 nm, preferably from about 3, to 21nm, preferably to about 10nm, more preferably to about 9nm.

The applicants have measured the Radioactive Dentine Abrasion (RDA) values of a number of silicas (15) having PAVs in the range 7.5 to 31 and have found a correlation coefficient of 0.91 (confidence 99%). PAVs of 8 and 15 correspond to mean RDAs of 55 and 109.

The silicas of the invention usually have a phase of alpha-cristobalite after firing at 1100°C.

The precipitated silicas of the invention are capable of providing satisfactory abrasion even at relatively low particle sizes i.e. 5 to 10 micron range. Abrasive materials can also be obtained at particle sizes beyond the stated upper limit but they are not suitable for use in toothpastes because of the unacceptable mouth feel of the coarser particles.

In general, characterisation of the pore structure of silicas containing higher levels of openness and wider pores by nitrogen adsorption techniques is not meaningful because this technique is useful only for pores up to about 60 nm diameter. To measure the full range of porosity present in such materials it is necessary to employ alternative procedures, for example oil absorption or mercury porosimetry. Since the products of this invention have some pore structure in excess of 60 nm it is necessary to define them by means of such techniques.

In the Examples the silicas having 70% transmission in the RI range 1.435 to 1.444 have a mercury intrusion volume above 0.5 $cm^3$/100g.

Literature

EPA 0227334 (Unilever) describes precipitated silicas having high absorbency, i.e. 110 to 180 $cm^3$/100g but the silicas of the present invention have enhanced clarity which allows their use in transparent toothpaste compositions.

Compared with the silicas described in EPA 0308165 (Unilever) the present silicas have lower abrasivity which allow their use at higher loadings in toothpaste formulations on a more flexible basis. These silicas are defined as having plastic abrasion values down to 15 with mean pore diameters in the range 3 to 8nm.

The use of Neosyl(R), a precipitated silica obtainable from Crosfield Chemicals of Warrington, England, is described in US 3864470 (Lever Bros Company). Neosyl, which is a mild polishing agent, would give excessive thickening, because of its higher structure, at the levels required to provide satisfactory cleaning.

The present silicas can be formulated into toothpaste compositions having a lower refractive index, i.e. 1.435 to 1.444 compared with the silicas described in GB 2146317 (Taki). These lower refractive index compositions are more economical to prepare since they have a higher water content in the formulation and therefore a corresponding lower amount of humectant e.g. Sorbitol, which is a more expensive component of the toothpaste formulation. The applicants have found these Taki silicas have plastic abrasion values and oil absorptions outside the ranges claimed in the present application.

Standard Procedures

The silicas of the invention are defined in terms of their physical and chemical properties. The standard test methods used to determine these properties are:-

i) Surface Area:

Surface area is determined by standard nitrogen adsorption methods of Brunauer, Emmett and Teller (BET) using a single point method with a Sorpty 1750 apparatus supplied by Carlo Erba company of Italy. The sample was outgassed under vacuum at 270°C for 1 hour before measurement.

ii) Oil Absorption:

The oil absorption is determined by the ASTM spatula rub-out method (American Society of Test Material Standards D, 281).

The test is based upon the principle of mixing linseed oil with a silica by rubbing with a spatula on a smooth surface until a stiff putty-like paste is formed which will not break or separate when it is cut with the spatula. The volume of oil used is then put into the following equation:-

$$\text{Oil absorption} = \frac{cm^3 \text{ oil absorption x 100}}{\text{wt. of silica sample in gms}}$$

$$= cm^3 \text{ oil/100g silica}$$

iii) Weight Mean Particle Size:

The weight mean particle size of the silicas was determined with the aid of a Malvern Particlesizer, Model 3600 E. This instrument, made by Malvern Instruments, Malvern, Worcestershire uses the principle of Fraunhöffer diffraction utilizing a low power He/Ne laser. Before measurement the sample was dispersed ultrasonically in water for a period of 7 minutes to form an aqueous suspension.

iv) Plastics Abrasion Value (PAV):

This test is based upon a toothbrush head brushing a Perspex plate in contact with a suspension of the silica in a sorbitol/glycerol mixture. Normally the slurry composition is as follows:

| | |
|---|---|
| Silica | 2.5 grams |
| Glycerol | 10.0 grams |
| Sorbitol | 23.0 grams |

All components are weighed into a beaker and dispersed for 2 minutes at 1500 rpm using a simple stirrer. A 110mm x 55mm x 3mm sheet of standard clear Perspex is used for the test, supplied by Imperial Chemical Industries PLC of England under code 000.

The test is carried out using a modified Wet Paint Scrub Tester produced by Research Equipment Limited, Wellington Road, Hampton Hill, Middlesex. The modification is to change the holder so that a toothbrush can be used instead of a paint brush. In addition a weight of 14 ozs (398g) is attached to the brush to force the brush onto the perspex plate.

A Galvanometer is calibrated using a 45° Plaspec gloss head detector and a standard (50% gloss) reflecting plate. The Galvanometer reading is adjusted to a value of 50 under these conditions. The reading of the fresh Perspex plate is then carried out using the same reflectance arrangement.

The fresh piece of Perspex is then fitted into a holder. Two mls of the dispersed silica, sufficient to lubricate fully the brushing stroke, is placed on the plate and the brush head lowered onto the plate. The machine is switched on and the plate subjected to three hundred strokes of the weighted brush head. The plate is removed from the holder and all the suspension is washed off. It is then dried and re-measured for its gloss value. The abrasion value is the difference between the unabraded value and the value after abrasion.

This test procedure, when applied to known abrasives, gave the following values:

|  | Plastics abrasion value |
| --- | --- |
| Calcium carbonate (15 micron) | 32 |
| Silica xerogel (10 micron) prepared by UK 1264292 method | 25 |
| Alumina trihydrate (Gibbsite) 15 micron | 16 |
| Calcium pyrophosphate (10 micron) | 14 |
| Dicalcium phosphate dihydrate (15 micron) | 7 |
| Neosyl[R] as used in US 3864470 (15 micron) | 8 |

v) Electrolyte Levels:

Sulphate is determined gravimetrically by hot water extraction of the silica, followed by precipitation as barium sulphate. Chloride is determined by hot water extraction of the silica, followed by titration with standard silver nitrate solution using potassium chromate as indicator (Mohr's method).

vi) Moisture Loss at 105°C:

Moisture loss is determined by the loss in weight of a silica when dried to constant weight in an electric oven at 105°C.

vii) Ignition Loss at 1000°C:

Ignition loss is determined by the loss in weight of a silica when ignited in a furnace at 1000°C to constant weight.

viii) pH:

This measurement is carried out on a 5% w/w suspension of the silica in boiled demineralised water ($CO_2$ free).

ix) Refractive index (RI)/transmission:

The sample of silica is dispersed in a range of Sorbitol syrup (70% Sorbitol)/water mixtures. After deaeration, usually 1 hour, the transmissions of the dispersions are determined using a spectrophotometer at 589 nm; water being used as blank. The refractive index of each dispersion is also measured using an Abbe refractometer.

A graphical representation of transmission plotted against refractive index allows the range of refractive indices over which the transmission exceeds 70% to be determined. The maximum transmission of the sample and the refractive index at which this is obtained can also be estimated from this graph.

The silicas of the invention are required to have a transmission of at least 80% at a point in the RI range of 1.435 to 1.456.

x) Viscosity determination:

Slurries of silica, at 10% and 20% w/w concentration, in 70% Sorbitol Syrup (E420) are prepared using a Heidolph stirrer at 1500 rpm for 10 minutes.

The viscosity of the slurry is determined @ $100s^{-1}$ shear rate using a Haake Rotovisco (R)RV12 @ 25°C.

The following conditions are used:-

Measuring head M500
Sensor system MVI
PG 142 Programme 1-3-1
Speed range 0-128 rpm

xi) Fluoride compatibility:

A sample of abrasive is slurried in a solution (23% w/v) of sodium fluoride for 1 hour at 60°C. The resultant slurry is centrifuged, the dear supernatant liquid is diluted 1:1 with a EDTA/TRIS buffer solution and the fluoride concentration is determined by direct potentiometry using a fluoride ion selective electrode in accordance with the method given in Textbook of Quantitative Chemistry by Vogel (5th Edition 1989) page 570 section 15. 16. The fluoride compatability is determined by comparison of the sample result with a fluoride standard solution.

xii) Mercury intrusion volume:

Mercury intrusion volumes are determined (in $cm^3/g$) by standard mercury intrusion procedures using a Micromeritics Autopore 9220 mercury porosimeter. The pore radius is calculated from the Washburn equation using values of surface tension for mercury of 485 dynes/cm and contact angle of 140°.

Prior to measurement the sample was outgassed at room temperature to a pressure of 50 microns of mercury. The mercury intrusion volume recorded is that occurring over the range of calculated pore diameters of 0.05 to 1.0 micron.

xiii) Mean pore diameter (MPD):

This parameter is related to the surface area and pore volume and, using a cylindrical pore model, is calculated for a silica product with the formula:

$$\text{MPD (nm)} = \frac{\text{pore volume } (cm^3 g^{-1}) \times 4000}{\text{surface area } (m^2 g^{-1})}$$

Pore volume is the mercury intrusion volume defined in (xii).

xiv) Crystal form after firing at 1100°C:

A sample of the silica is fired in an electric muffle furnace for one hour at 1100°C. The treated sample is allowed to cool and the crystal structure present identified from the trace obtained from an x-ray diffractometer.

Specific description of the invention

Examples of the preparation of precipitated silicas will now be given to illustrate but not limit the invention.

Mixing is an important feature in the reaction of silicate and sulphuric acid. Consequently fixed specifications as listed in Chemineer Inc. Chem Eng. April 26th 176 Pages 102-110 have been used to design the baffled heated stirred reaction vessels. Whilst the turbine design is optional to the mixing geometry, a 6-bladed 30° pitched bladed unit has been chosen for our experiments in order to ensure maximum mixing effectiveness with minimum shear. Shear has been supplied to the reactant mixture by circulating the contents of the reaction vessel through an external high shear mixer (Silverson) containing a square hole high shear screen throughout the simultaneous addition of silicate and acid; the energy input being commensurate with the volume flow and number of recirculations required as specified by the manufacturer.

The solutions used in the process were as follows:-

**EP 0 495 039 B2**

i) Sodium silicate solutions having a $SiO_2$:$Na_2O$ ratio in the range of 3.1 to 3.4:1.

ii) A sulphuric acid solution of specific gravity 1.10(15.0% W/W solution) to 1.15 (21.4% W/W solution).

iii) An electrolyte solution as defined in each example

The following procedure was adopted in the preparation of the precipitated silicas. Values of reactant concentrations and volumes, and reaction temperatures are given in Table 1.

(A) litres of water were placed in the vessel together with (B) litres of electrolyte solution and (C) litres of the sodium silicate solution. This mixture was then stirred and heated to (E)°C.

The sodium silicate ((D) litres) and sulphuric acid ((F) litres) solutions were then added simultaneously over a period of about 20 minutes with stirring while maintaining the temperature at (E)°C. The flow rates of the silicate and acid solutions were uniform throughout the addition period to ensure that a constant pH was maintained in the vessel. Sulphuric acid solution was then added over a period of 10 minutes with continued mixing to reduce the pH of the liquid to the range of 2.5, usually 3.0, to 4.0. During this addition of acid the temperature was maintained. The resultant slurry was then filtered and washed with water to remove excess electrolyte. Typically, for a toothpaste application, the residual electrolyte would be less than 2% on a dry weight basis.

After washing, the filter cake in each example was flash dried and comminuted to the desired particle size range.

The precipitated silica obtained had the properties, expressed on a dry weight basis, as listed in Table II.

The amorphous precipitated silicas prepared as described provided satisfactory cleaning properties for the transparent toothpastes in which they were incorporated. The toothpastes had commercially suitable properties for stability and usage. A typical formulation using a silica of this invention is listed below.

Transparent Gel Toothpaste

|  | % |
| --- | --- |
| Sorbosil TC15 | 6.0 |
| Silica of invention | 16.0 |
| Sodium Carboxymethyl Cellulose | 0.7 |
| Sorbitol, 70% non-crystallisable | 60.0 |
| Polyethylene Glycol 1500 | 5.0 |
| Sodium Lauryl Sulphate | 1.5 |
| Sodium Monofluoro-phosphate | 0.8 |
| Flavour | 1.0 |
| Saacharin | 0.2 |
| Colour, Blue, CI42090 Water & minor ingredients | 0.002 to 100 |
| Properties - Initial Density gml$^{-1}$ (25°C) | 1.37 |

Sorbosil TC15 is a thickening silica obtainable from Crosfield Chemicals of Warrington, England.

TABLE I

| Example No. | 1 | 2 | 3 |
| --- | --- | --- | --- |
| Vessel capacity (litres) | 64 | 64 | 325 |
| Water volume (A) (litres) | 13.5 | None | 106 |
| Electrolyte used | NaCl | $Na_2SO_4$ | NaCl |
| Concn. of electrolyte (%w/w) | 25 | 6.9 | 25 |
| Vol. of electrolyte (B) (litres) | 1.3 | 17.4 | 15.2 |
| Silicate ratio $SiO_2$/$Na_2O$ by weight | 3.30 | 3.25 | 3.31 |
| $SiO_2$ Concentration in sodium silicate (%w/w) | 17.5 | 17.5 | 16.7 |
| Silicate volume (C) (litres) | 0.1 | 0.1 | 1.3 |
| Silicate volume (D) (litres) | 10.7 | 9.2 | 124.3 |

TABLE I (continued)

| Example No. | 1 | 2 | 3 |
|---|---|---|---|
| Acid concentration (%w/w) | 16.3 | 16.0 | 17.3 |
| Acid volume (F) (litres) | 4.2 | 3.3 | 45.7 |
| Temperature °C (E) | 80 | 98 | 85 |

TABLE 2

| Example No. | 1 | 2 | 3 |
|---|---|---|---|
| Mercury intrusion vol ($cm^3$/g) | 0.61 | 0.57 | 1.51 |
| Mean pore diameter (nm) | 7.5 | 4.7 | 20.5 |
| Surface area ($m^2g^{-1}$) | 327 | 480 | 293 |
| Oil absorption ($cm^3$/100g) | 130 | 110 | 158 |
| Weight mean particle size (micron) | 11.2 | 14.5 | 8.5 |
| Plastic abrasion value | 12 | 11 | 12 |
| Max % transmission | 90 | 83 | 89 |
| at refractive index | 1.442 | 1.440 | 1.444 |
| Available fluoride (%) | 96.6 | 93.9 | 94.6 |
| Electrolyte level ($SO_4$=) (% W/W) | 0.08 | 0.13 | 0.19 |
| Electrolyte level ($Cl^-$) (% W/W) | 0.06 | 0.03 | 0.03 |
| Moisture loss @ 105°C (%) | 3.2 | 4.1 | 5.0 |
| Ignition loss @ 1000°C (% W/W) | 8.6 | 9.0 | 10.3 |
| pH | 7.1 | 6.7 | 6.9 |

TABLE 3

| Viscosity of silicas dispersed in 70% Sorbitol Syrup (E420) mPas at $100s^{-1}$ | | | |
|---|---|---|---|
| | OA | 10% w/w Silica | 20% w/w Silica |
| Example 3 Silica | 158 | 161 | 1127 |
| Low Structure commercially available silica abrasive | 110 | 186 | 303 |
| Medium Structure commercially available silica (Neosyl$^R$) | 170 | 290 | 1406 |
| High Structure commercially available silica | 200 | 1448 | Too high to measure on this sensor |

This demonstrates the silicas of this invention that have higher oil absorption (OA) give, at high concentration in sorbitol syrup, a higher level of thickening than that usually associated with a low structure commercially available abrasive silica.

**Claims**

1. An amorphous precipitated silica having:

   i) a surface area in the range from 250 to 600 $m^2$/g,
   ii) an oil absorption (using linseed oil) from 90, preferably from 110, to 160 $cm^3$/100g,
   iii) a weight mean particle size in the range from 5 to 20 microns,
   iv) a plastics abrasion value in the range from 8 to 14,
   v) a transmission of at least 80% within the refractive index range of 1.435 to 1.444, and
   vi) a mean pore diameter in the range from 1.5 to 21 nm.

**2.** A silica according to claim 1 wherein the surface area is at least 300 m$^2$/g.

**3.** A silica according to claim 1 or 2 wherein the surface area is not more than 550 m$^2$/g.

**4.** A silica according to claim 1, 2 or 3 wherein the oil absorption is not more that 140 cm$^3$/100g.

**5.** A silica according to claim 1, 2, 3 or 4 wherein the plastics abrasion value is at least 10.

**6.** A silica according to any preceding claim wherein the mean pore diameter is at least 3 nm.

**7.** A silica according to any preceding claim wherein the mean pore diameter is not more than 10 nm.

**8.** A silica according to any preceding claim wherein the weight mean particle size is not more than 15 microns.

**9.** A silica according to any preceding claim wherein the phase after firing at 1100°C is alpha-cristobalite.

**10.** A silica according to any preceding claim having a moisture content of less than 25%, preferably less than 15% w/w.

**11.** A silica according to any preceding claim in which the total of soluble calcium, magnesium and aluminium is not more than 500 ppm and the silica has fluoride compatibility above 90%.

**12.** A toothpaste composition containing from 5% to 50% by weight, preferably up to 30%, of an amorphous precipitated silica defined in any preceding claim.

**Patentansprüche**

**1.** Amorphe, gefällte Kieselsäure, die aufweist:

i) eine spezifische Oberfläche im Bereich von 250 bis 600 m$^2$/g,
ii) eine Ölabsorption (unter Verwendung von Leinsamenöl) von 90, vorzugsweise von 110, bis 160 cm$^3$/100 g,
iii) eine gewichtsgemittelte Teilchengröße im Bereich von 5 bis 20 µm,
iv) einen Kunststoff-Schleifwert im Bereich von 8 bis 14,
v) eine Transmission von mindestens 80 % innerhalb des Brechungszahlbereiches von 1,435 bis 1,444 und
vi) einen mittleren Porendurchmesser im Bereich von 1,5 bis 21 nm.

**2.** Kieselsäure nach Anspruch 1, worin die spezifische Oberfläche mindestens 300 m$^2$/g beträgt.

**3.** Kieselsäure nach Anspruch 1 oder 2, worin die spezifische Oberfläche nicht mehr als 550 m$^2$/g beträgt.

**4.** Kieselsäure nach Anspruch 1, 2 oder 3, worin die Ölabsorption nicht mehr als 140 cm$^3$/100 g beträgt.

**5.** Kieselsäure nach Anspruch 1, 2, 3 oder 4, worin der Kunststoff-Schleifwert mindestens 10 beträgt.

**6.** Kieselsäure nach irgendeinem vorhergehenden Anspruch, worin der mittlere Porendurchmesser mindestens 3 nm beträgt.

**7.** Kieselsäure nach irgendeinem vorhergehenden Anspruch, worin der mittlere Porendurchmesser nicht mehr als 10 nm beträgt.

**8.** Kieselsäure nach irgendeinem vorhergehenden Anspruch, worin die gewichtsgemittelte Teilchengröße nicht mehr als 15 µm beträgt.

**9.** Kieselsäure nach irgendeinem vorhergehenden Anspruch, worin die Phase nach Brennen bei 1100°C alpha-Cristobalit ist.

**10.** Kieselsäure nach irgendeinem vorhergehenden Anspruch mit einem Feuchtigkeitsgehalt von weniger als 25 %, vorzugsweise weniger als 15 %, Gew./Gew.

**11.** Kieselsäure nach irgendeinem vorhergehenden Anspruch, in welcher die Gesamtheit von löslichem Calcium, Magnesium und Aluminium nicht mehr als 500 ppm beträgt und die Kieselsäure eine Fluoridkompatibilität von mehr als 90 % aufweist.

**12.** Zahnpastazusammensetzung, die 5 bis 50 Gew.-%, vorzugsweise bis zu 30 %, einer in einem vorhergehenden Anspruch definierten amorphen, gefällten Kieselsäure enthält.

**Revendications**

**1.** Silice précipitée amorphe ayant :

   i) une surface spécifique comprise entre 250 et 600 m$^2$/g,
   ii) une absorption d'huile (en utilisant de l'huile de lin) comprise entre 90, de préférence entre 110, et 160 cm$^3$/100 g,
   iii) une dimension de particule moyenne en poids comprise entre 5 et 20 micromètres,
   iv) une valeur d'abrasion des matières plastiques comprise entre 8 et 14,
   v) une transmission d'au moins 80% dans l'intervalle d'indices de réfraction de 1,435 et 1,444, et
   vi) un diamètre moyen des pores compris entre 1,5 et 21 nm.

**2.** Silice conforme à la revendication 1, dans laquelle la surface spécifique est d'au moins 300 m$^2$/g.

**3.** Silice conforme à la revendication 1 ou la revendication 2, dans laquelle la surface spécifique est inférieure à 550 m$^2$/g.

**4.** Silice conforme à la revendication 1, 2 ou 3, dans laquelle l'absorption d'huile est inférieure à 140 cm$^3$/100 g.

**5.** Silice conforme à la revendication 1, 2, 3 ou 4, dans laquelle la valeur d'abrasion des matières plastiques est d'au moins 10.

**6.** Silice conforme à l'une quelconque des revendications précédentes, dans laquelle le diamètre moyen des pores est d'au moins 3 nm.

**7.** Silice conforme à l'une quelconque des revendications précédentes, dans laquelle le diamètre moyen des pores est inférieur à 10 nm.

**8.** Silice conforme à l'une quelconque des revendications précédentes, dans laquelle la dimension moyenne de particule en poids est inférieure à 15 micromètres.

**9.** Silice conforme à l'une quelconque des revendications précédentes, dans laquelle la phase après chauffage à 1100°C est l'alpha-cristobalite.

**10.** Silice conforme à l'une quelconque des revendications précédentes, ayant une teneur en humidité inférieure à 25%, de préférence inférieure à 15% p/p.

**11.** Silice conforme à l'une quelconque des revendications précédentes, dans laquelle la quantité totale de calcium, magnésium et aluminium solubles est inférieure à 500 ppm, la silice ayant une compatibilité avec les fluorures supérieure à 90%.

**12.** Composition de pâte dentifrice contenant entre 5% et 50% en poids, de préférence jusqu'à 30%, d'une silice précipitée amorphe définie dans l'une quelconque des revendications précédentes.